# EUROPEAN PATENT APPLICATION

(11) **EP 4 667 925 A1**
(43) Date of publication of application: **24.12.2025**
(21) Application number: 24778089.3
(22) Date of filing: 27.03.2024
(51) Int. Cl.: G01N 27/327, A61N 1/05, A61B 5/25, H05K 3/10

(54) **PREPARATION METHOD FOR BIOSENSOR, AND BIOSENSOR AND RELATED PRODUCT THEREOF**

(30) Priority: 30.03.2023 CN 202310364689
(71) Applicant: Huawei Technologies Co., Ltd., Shenzhen, Guangdong 518129 (CN)
(72) Inventor: PAN, Xiang, Shenzhen, Guangdong 518129 (CN); CHEN, Liangru, Shenzhen, Guangdong 518129 (CN); YANG, Yingxi, Shenzhen, Guangdong 518129 (CN); DING, Zhaoyi, Shenzhen, Guangdong 518129 (CN); GUO, Jutian, Shenzhen, Guangdong 518129 (CN)
(74) Representative: Maiwald GmbH
(86) International application number: PCT/CN2024/084198
(87) International publication number: WO 2024/199303

(57) **Abstract**

This application provides a preparation method for a biosensor, a biosensor, and a related product thereof. The preparation method for a biosensor includes: providing a first circuit board, where the first circuit board includes a first insulation layer and a first metal layer stacked on the first insulation layer; forming a first cable trough on the first insulation layer through laser etching, where a part of the first metal layer is exposed through the first cable trough; forming a first working electrode on the exposed part of the first metal layer; forming a second insulation layer on a surface that is of the first working electrode and that is away from the first metal layer and a surface that is of the first insulation layer and that is away from the first metal layer; removing the first metal layer; and forming a first active substance layer on a surface that is of the first working electrode and that is away from the second insulation layer. According to the preparation method for a biosensor in this application, a biosensor with high precision and high consistency can be obtained when a technological process is simplified, to improve a production capacity and reduce manufacturing cost.

## Description

This application claims priority to Chinese Patent Application No. 202310364689.5, filed with the China National Intellectual Property Administration on March 30, 2023, and entitled "PREPARATION METHOD FOR BIOSENSOR, BIOSENSOR, AND RELATED PRODUCT THEREOF", which is incorporated herein by reference in its entirety.

### TECHNICAL FIELD

This application relates to the field of biosensors, and in particular, to a preparation method for a biosensor, a biosensor, and a related product thereof.

### BACKGROUND

Electrode-based biosensors implant electrodes into human bodies for reaction with target analytes in the human bodies, to obtain corresponding detection information. The electrode-based biosensors are increasingly widely used. At present, the electrode-based biosensors on the market are generally prepared by using a semiconductor process or a printing process. However, preparation using the semiconductor process has high cost and a complex technological process, and is unsuitable for mass production. Although preparation using the printing process has lower cost, precision and consistency of prepared biosensors are usually low.

### SUMMARY

An implementation of this application provides a preparation method for a biosensor, a biosensor prepared according to the preparation method, and a related product thereof. According to the preparation method for a biosensor in this application, a biosensor with high precision and high consistency can be obtained when a technological process is simplified, to improve a production capacity and reduce manufacturing cost.

According to a first aspect, a preparation method for a biosensor is provided. The preparation method for a biosensor includes: providing a first circuit board, where the first circuit board includes a first insulation layer and a first metal layer stacked on the first insulation layer; forming a first cable trough on the first insulation layer through laser etching, where a part of the first metal layer is exposed through the first cable trough; forming a first working electrode on the exposed part of the first metal layer; forming a second insulation layer on a surface that is of the first working electrode and that is away from the first metal layer and a surface that is of the first insulation layer and that is away from the first metal layer; removing the first metal layer; and forming a first active substance layer on a surface that is of the first working electrode and that is away from the second insulation layer.

It may be understood that, according to the biosensor in this implementation, the first cable trough is first etched by using a high-precision laser, and then the first working electrode is formed in the first cable trough. In this way, precision of the formed first cable trough is high, so that precision of the subsequently formed first working electrode is also high. In addition, compared with a biosensor prepared by using a printing process, the biosensor prepared according to the preparation method in this implementation has higher consistency and higher precision.

In a possible implementation, in the step of forming the first working electrode on the exposed part of the first metal layer, the preparation method further includes: forming the first working electrode by using an electroplating process on the exposed part of the first metal layer.

It may be understood that, the preparation method for a biosensor provided in this implementation is different from a manner in which a biosensor is prepared by using a semiconductor front-end-of-line or a micro-electro-mechanical system-like process in that preparation is performed based on a conventional flexible circuit board process, so that the biosensor can be directly produced in a conventional FPC factory, to facilitate mass production of the biosensor and improve a production capacity. In addition, the preparation method in this implementation may be compatible with a current FPC process. Preparation is directly performed on a circuit board by using both a laser etching process and an electroplating process, so that a technological process of the preparation method is simpler. In this way, cost is lower, and production efficiency is effectively improved. In addition, compared with a biosensor prepared by using a printing process, the biosensor prepared according to the preparation method in this implementation has higher consistency and higher precision. In other words, according to the preparation method for a biosensor in this implementation, a biosensor with high precision and high consistency can be obtained when a technological process is simplified, to improve a production capacity and reduce manufacturing cost.

In a possible implementation, after the step of removing the first metal layer, and before the step of forming the first active substance layer on the surface that is of the first working electrode and that is away from the second insulation layer, the preparation method further includes: forming a third insulation layer on a surface that is of the first insulation layer and that is away from the second insulation layer and the surface that is of the first working electrode and that is away from the second insulation layer; and forming a first exposure groove on the third insulation layer, where a part of the first working electrode is exposed through the first exposure groove.

It may be understood that, in the preparation method in this implementation, the third insulation layer is further formed on the surface that is of the first insulation layer and that is away from the second insulation layer and the surface that is of the first working electrode and that is away from the second insulation layer, and the first exposure groove is formed on the third insulation layer to expose at least a part of the first working electrode. In this way, the formed first exposure groove may limit the formed first active substance layer in a horizontal direction, so that an area and a shape of the first active substance layer can be accurately controlled, to improve detection precision of the biosensor. In addition, the third insulation layer may further protect a part that is of the first working electrode and that is not exposed through the first exposure groove, so that direct contact between the part that is of the first working electrode and that is not exposed through the first exposure groove and an external environment is avoided, to avoid a fault such as a short circuit of the biosensor, and structural stability of the biosensor can be improved, to improve a service life of the biosensor.

In a possible implementation, in the step of forming the first working electrode on the exposed part of the first metal layer, the preparation method further includes: forming the first working electrode on an exposed part of a first surface of the first metal layer, where the first surface is a surface that is of the first metal layer and that is close to the first insulation layer; and after the step of forming the first working electrode on the exposed part of the first metal layer, and before the step of forming the second insulation layer on the surface that is of the first working electrode and that is away from the first metal layer and the surface that is of the first insulation layer and that is away from the first metal layer, the preparation method further includes: forming a first counter electrode and a first reference electrode on the exposed part of the first surface, where the first working electrode, the first counter electrode, and the first reference electrode are disposed on the first surface in a staggered manner. In this way, the first working electrode, the first counter electrode, and the first reference electrode cooperate with each other, which helps improve detection precision of the biosensor. In addition, the first working electrode, the first counter electrode, and the first reference electrode are located on a same plane, so that an overall thickness of the biosensor can be greatly reduced when good electrochemical performance is ensured. In this way, a thin design of the biosensor is implemented, to improve wearing comfort of the biosensor, so that user experience is improved.

In a possible implementation, after the step of forming the first working electrode on the exposed part of the first metal layer, and before the step of forming the second insulation layer on the surface that is of the first working electrode and that is away from the first metal layer and the surface that is of the first insulation layer and that is away from the first metal layer, the preparation method further includes: forming a first blank electrode on the exposed part of the first surface, where the first blank electrode and the first working electrode, the first counter electrode, and the first reference electrode are disposed on the first surface in a staggered manner.

It may be understood that the biosensor prepared according to the preparation method in this implementation is a four-electrode system that includes the first working electrode, the first counter electrode, the first reference electrode, and the first blank electrode. In this way, the first blank electrode is disposed, to effectively reduce interference of signal background noise, so that detection precision of the biosensor is improved. In addition, the first working electrode, the first counter electrode, the first reference electrode, and the first blank electrode are located on a same plane, so that an overall thickness of the biosensor can be greatly reduced when good electrochemical performance is ensured. In this way, a thin design of the biosensor is implemented, to improve wearing comfort of the biosensor, so that user experience is improved.

In a possible implementation, a material of the first working electrode is platinum, gold, or palladium. In this way, the material of the first working electrode is a biocompatible material. When the biosensor is used in human body detection, the first working electrode does not cause the human body to generate a rejection reaction. This helps improve detection safety.

In a possible implementation, after the step of forming the first working electrode on the exposed part of the first metal layer, and before the step of forming the second insulation layer on the surface that is of the first working electrode and that is away from the first metal layer and the surface that is of the first insulation layer and that is away from the first metal layer, the preparation method further includes: forming a first trace and a first pad on the exposed part of the first metal layer, where the first working electrode is connected to the first pad through the first trace. In this way, the first working electrode may be electrically connected to the first pad through the first trace, and is electrically connected to an external component of the biosensor through the first pad.

In a possible implementation, after the step of forming the second insulation layer on the surface that is of the first working electrode and that is away from the first metal layer and the surface that is of the first insulation layer and that is away from the first metal layer, and before the step of removing the first metal layer, the preparation method further includes: providing a second circuit board, where the second circuit board includes a fourth insulation layer and a second metal layer stacked on the fourth insulation layer; forming a second cable trough on the fourth insulation layer through laser etching, where a part of the second metal layer is exposed through the second cable trough; forming a second working electrode on the exposed part of the second metal layer; forming a fifth insulation layer on a surface that is of the second working electrode and that is away from the second metal layer and a surface that is of the fourth insulation layer and that is away from the second metal layer; and placing the first circuit board on the second circuit board, so that the second insulation layer is connected to the fifth insulation layer. In this way, the second circuit board is placed on the first circuit board, and the second insulation layer is connected to the fifth insulation layer, which facilitates subsequent preparation.

In a possible implementation, in the step of removing the first metal layer, the preparation method further includes: removing the second metal layer; and in the step of forming the first active substance layer on the surface of the first working electrode, the preparation method further includes: forming a second active substance layer on a surface of the second working electrode.

It may be understood that different working electrodes (namely, the first working electrode and the second working electrode) are respectively disposed on two sides of the biosensor prepared according to the preparation method in this implementation. In this way, when the first working electrode and the second working electrode react with a same target analyte, a linear detection range of the biosensor can be effectively expanded. When the first working electrode and the second working electrode react with different target analytes respectively, detection efficiency of the biosensor can be effectively improved, so that one biosensor can perform accurate detection on a plurality of target analytes simultaneously. Second, the preparation method in this implementation may be compatible with a current FPC process. Preparation is directly performed on a circuit board by using both a laser etching process and an electroplating process, so that an overall technological process is simpler. In this way, cost is lower, and production efficiency is effectively improved. In addition, the biosensor prepared according to the preparation method in this implementation has higher consistency and higher precision. In other words, according to the preparation method for a biosensor in this implementation, a biosensor with high precision, high consistency, and high detection efficiency can be obtained when a technological process is simplified, to improve a production capacity and reduce manufacturing cost.

In a possible implementation, after the step of forming the second working electrode on the exposed part of the second metal layer, and before the step of forming the fifth insulation layer on the surface that is of the second working electrode and that is away from the second metal layer and that is of the fourth insulation layer and that is away from the second metal layer, the preparation method further includes: forming a second trace and a second pad on the exposed part of the second metal layer, where the second working electrode is connected to the second pad through the second trace. In this way, the second working electrode may be electrically connected to the second pad through the second trace, and is electrically connected to an external component of the biosensor through the second pad.

In a possible implementation, after the step of forming the first cable trough on the first insulation layer through laser etching, and before the step of forming the first working electrode on the exposed part of the first metal layer, the preparation method further includes: forming a third cable trough on the first insulation layer through laser etching, where a part of the first metal layer is exposed through the third cable trough, and the third cable trough is separated from the first cable trough by the first insulation layer;
after the step of forming the first working electrode on the exposed part of the first metal layer, and before the step of forming the second insulation layer on the surface that is of the first working electrode and that is away from the first metal layer and the surface that is of the first insulation layer and that is away from the first metal layer, the preparation method further includes: forming a third pad on the first metal layer exposed through the third cable trough; and
after the step of removing the first metal layer, and before the step of forming the first active substance layer on the surface that is of the first working electrode and that is away from the second insulation layer, the preparation method further includes: forming a first channel through laser etching sequentially performed on the third pad, the second insulation layer, and the fifth insulation layer, where a part of the second pad is exposed through the first channel; and forming a third trace on an exposed part of a surface of the second pad, where the first channel is filled with the third trace, and the second pad is connected to the third pad through the third trace.

It may be understood that, in this implementation, the third pad is further formed when the first working electrode, the first trace, and the first pad are formed, and is separated from the first pad by the first insulation layer. The third pad and the second pad may be symmetrically disposed relative to the second insulation layer and the fifth insulation layer. Then, the first channel is formed through laser etching sequentially performed on the third pad, the second insulation layer, and the fifth insulation layer. Then, the third trace is formed in the first channel, so that the second pad may be connected to the third pad through the third trace. In this way, the first working electrode may be electrically connected to an external environment through the first pad, the second electrode may be electrically connected to the external environment through the third pad, and the first pad and the third pad are located on a same plane. In this way, when a subsequently prepared biosensor is soldered or connected to another device such as a connector, connection needs to be performed on only one side of the biosensor, and does not need to be separately performed on two sides of the biosensor, so that a connection process is more convenient. In addition, when the biosensor needs to be connected to the connector, the connector needs to be connected to only one side of the biosensor, so that an overall thickness of the biosensor and the connector is thin. This helps improve user experience.

In a possible implementation, in the step of forming the first active substance layer on the surface that is of the first working electrode and that is away from the second insulation layer, the preparation method further includes: forming a first connection layer on the surface that is of the first working electrode and that is away from the second insulation layer; and forming the first active substance layer on a surface that is of the first connection layer and that is away from the first working electrode. In this way, the first connection layer is disposed to be connected between the first active substance layer and the first working electrode, so that a bonding force between the first active substance layer and the first working electrode can be effectively increased, to improve overall structural stability of the biosensor.

In a possible implementation, after the step of forming the first active substance layer on the surface that is of the first working electrode and that is away from the second insulation layer, the preparation method further includes: forming a first limiting layer on a surface that is of the first active substance layer and that is away from the first working electrode.

It may be understood that, in the biosensor prepared according to the preparation method in this implementation, the first limiting layer is further disposed on the surface of the first active substance layer. In this way, the first limiting layer may restrict the target analyte from entering the first active substance layer, so that a linear detection range of the biosensor can be expanded. In addition, a material of the first limiting layer is a biocompatible material, so that one end that is of the biosensor and that is close to the first working electrode can be implanted into a human body, and no rejection reaction occurs when the end that is of the biosensor and that is close to the first working electrode reacts with the target analyte in the human body. In this way, the biosensor can be better used in human body detection.

According to a second aspect, a biosensor is provided. The biosensor is prepared according to the foregoing preparation method. It may be understood that, compared with a biosensor prepared by using a printing process, the biosensor prepared according to the foregoing preparation method has higher consistency and higher precision.

According to a third aspect, a biosensor is provided. The biosensor includes a first insulation layer, a second insulation layer, a first working electrode, and a first active substance layer. The first insulation layer is stacked on the second insulation layer. The first insulation layer is provided with a first cable trough. The first working electrode is disposed in the first cable trough. The first active substance layer is disposed on a surface that is of the first working electrode and that faces away from the second insulation layer. The first cable trough is formed through laser etching. It may be understood that the first working electrode of the biosensor in this implementation is disposed in the first cable trough. The first cable trough is formed through laser etching. In this way, precision of the first cable trough is high, so that precision of the first working electrode disposed in the first cable trough is also high. This helps obtain a biosensor with high precision and high consistency.

In a possible implementation, a radial width of the first cable trough is within a range of 10 micrometers to 100 micrometers. In this way, the radial width of the first cable trough is within the range of 10 micrometers to 100 micrometers, so that a radial width of the first working electrode disposed in the first cable trough is also within the range of 10 micrometers to 100 micrometers. In this way, precision of the first working electrode is high. This helps obtain a biosensor with high precision and high consistency.

In a possible implementation, the first working electrode is formed by using an electroplating process. It may be understood that, the first working electrode in this implementation is formed by using the electroplating process. In this way, the biosensor may be prepared based on a conventional flexible circuit board process, so that the biosensor can be directly produced in a conventional FPC factory, to facilitate mass production of the biosensor and improve a production capacity. In addition, the biosensor in this implementation may be compatible with a current FPC process, and may be directly prepared on a circuit board by using both a laser etching process and an electroplating process. In this way, a technological process is simpler, so that cost is lower, and production efficiency is effectively improved. In other words, the biosensor in this implementation may be prepared by using both the laser etching process and the electroplating process. A preparation process of the biosensor is simpler, and the prepared biosensor has high precision and high consistency. This helps improve detection precision of the biosensor.

In a possible implementation, the biosensor further includes a first connection layer. The first connection layer is connected between the first active substance layer and the first working electrode. In this way, the first connection layer is disposed to be connected between the first active substance layer and the first working electrode, so that a bonding force between the first active substance layer and the first working electrode can be effectively increased, to improve overall structural stability of the biosensor.

In a possible implementation, a material of the first connection layer is graphene, carbon nanotube, or carbon. In this way, the first connection layer may be more firmly connected between the first active substance layer and the first working electrode, to help improve structural stability of the biosensor.

In a possible implementation, the biosensor further includes a third insulation layer. The third insulation layer is stacked on a surface that is of the first insulation layer and that faces away from the second insulation layer. The third insulation layer is provided with a first exposure groove. A part of the first working electrode is exposed through the first exposure groove.

It may be understood that, the third insulation layer is formed on the surface that is of the first insulation layer and that is away from the second insulation layer and the surface that is of the first working electrode and that is away from the second insulation layer, and the first exposure groove is formed on the third insulation layer to expose at least a part of the first working electrode. In this way, the formed first exposure groove may limit the formed first active substance layer in a horizontal direction, so that an area and a shape of the first active substance layer can be accurately controlled, to improve detection precision of the biosensor. In addition, the third insulation layer may further protect a part that is of the first working electrode and that is not exposed through the first exposure groove, so that direct contact between the part that is of the first working electrode and that is not exposed through the first exposure groove and an external environment is avoided, to avoid a fault such as a short circuit of the biosensor, and structural stability of the biosensor can be improved, to improve a service life of the biosensor.

In a possible implementation, the biosensor further includes a fourth insulation layer, a fifth insulation layer, a second working electrode, and a second active substance layer. The fourth insulation layer is stacked on the fifth insulation layer, and the fifth insulation layer is connected to the second insulation layer. The fourth insulation layer is provided with a second cable trough. The second working electrode is disposed in the second cable trough. The second active substance layer is disposed on a surface that is of the second working electrode and that faces away from the fifth insulation layer.

It may be understood that different working electrodes (namely, the first working electrode and the second working electrode) are respectively disposed on two sides of the biosensor in this implementation. In this way, when the first working electrode and the second working electrode react with a same target analyte, a linear detection range of the biosensor can be effectively expanded. When the first working electrode and the second working electrode react with different target analytes respectively, detection efficiency of the biosensor can be effectively improved, so that one biosensor can perform accurate detection on a plurality of target analytes simultaneously.

In a possible implementation, the biosensor further includes a first pad and a second pad. The first pad is disposed in the first cable trough. The first pad is electrically connected to the first working electrode. The second pad is disposed in the second cable trough. The second pad is electrically connected to the second working electrode. The biosensor further includes a third pad and a third trace. The first insulation layer is further provided with a third cable trough. The third cable trough and the first cable trough are spaced apart. The third pad is disposed in the third cable trough. The biosensor is further provided with a first channel. The first channel penetrates through the third pad, the second insulation layer, and the fifth insulation layer. The third trace is disposed in the first channel, and is electrically connected to the second pad and the third pad.

It may be understood that, the first pad and the third pad of the biosensor in this implementation are located on a same side of the biosensor, and the third trace that is electrically connected between the second pad and the third pad is disposed, so that the second working electrode may be electrically connected to an external component of the biosensor through the third pad. In this way, when the biosensor is soldered or connected to another device such as a connector, connection needs to be performed on only one side of the biosensor, and does not need to be separately performed on two sides of the biosensor, so that a connection process is more convenient. In addition, when the biosensor needs to be connected to the connector, the connector needs to be connected to only one side of the biosensor, so that an overall thickness of the biosensor and the connector is thin. This helps improve user experience.

According to a fourth aspect, a detection device is provided. The detection device includes a controller, a connector, and the foregoing biosensor. The biosensor is electrically connected to the controller through the connector. The controller is configured to receive detection information of the biosensor. It may be understood that the first working electrode of the biosensor in the detection device in this implementation is disposed in the first cable trough. The first cable trough is formed through laser etching. In this way, precision of the first cable trough is high, so that precision of the first working electrode disposed in the first cable trough is also high. This helps obtain a biosensor with high precision and high consistency, and helps obtain a detection device with high precision.

According to a fifth aspect, a detection system is provided. The detection system includes an intelligent terminal and the foregoing detection device. The intelligent terminal is in communication connection with the detection device. It may be understood that, the detection device in the detection system in this implementation has high precision. This helps obtain a detection system with high precision.

### BRIEF DESCRIPTION OF DRAWINGS

To describe the technical solutions in implementations of this application or in the background more clearly, the following describes the accompanying drawings for describing the implementations of this application or the background.
FIG. 1 is a schematic flowchart of a preparation method for a biosensor in an implementation according to an implementation of this application;
FIG. 2 is a diagram of a preparation process of the preparation method shown in FIG. 1 in some implementations;
FIG. 3 is a diagram of a preparation process of the preparation method shown in FIG. 1 in some implementations;
FIG. 4 is a diagram of a preparation process of the preparation method shown in FIG. 1 in some implementations;
FIG. 5 is a diagram of a preparation process of the preparation method shown in FIG. 1 in some implementations;
FIG. 6 is a diagram of a preparation process of the preparation method shown in FIG. 1 in some implementations;
FIG. 7 is a diagram of a preparation process of the preparation method shown in FIG. 1 in some implementations;
FIG. 8 is a diagram of a preparation process of the preparation method shown in FIG. 1 in some implementations;
FIG. 9 is a diagram of a preparation process of the preparation method shown in FIG. 1 in some implementations;
FIG. 10 is a diagram of a preparation process of the preparation method shown in FIG. 1 in some implementations;
FIG. 11 is a diagram of a preparation process of the preparation method shown in FIG. 1 in some implementations;
FIG. 12 is a diagram of a preparation process of the preparation method shown in FIG. 1 in some implementations;
FIG. 13 is a diagram of a preparation process of the preparation method shown in FIG. 1 in some implementations;
FIG. 14 is a diagram of a preparation process of the preparation method shown in FIG. 1 in some implementations;
FIG. 15 is a diagram of a preparation process of the preparation method shown in FIG. 1 in some implementations;
FIG. 16A and FIG. 16B are a schematic flowchart of the preparation method for a biosensor shown in FIG. 1 in another implementation;
FIG. 17 is a diagram of a preparation process of the preparation method for a biosensor shown in FIG. 16A and FIG. 16B in some implementations;
FIG. 18 is a diagram of a preparation process of the preparation method for a biosensor shown in FIG. 16A and FIG. 16B in some implementations;
FIG. 19 is a diagram of a preparation process of the preparation method for a biosensor shown in FIG. 16A and FIG. 16B in some implementations;
FIG. 20 is a diagram of a preparation process of the preparation method for a biosensor shown in FIG. 16A and FIG. 16B in some implementations;
FIG. 21 is a diagram of a preparation process of the preparation method for a biosensor shown in FIG. 16A and FIG. 16B in some implementations;
FIG. 22 is a diagram of a preparation process of the preparation method for a biosensor shown in FIG. 16A and FIG. 16B in some implementations;
FIG. 23 is a diagram of a preparation process of the preparation method for a biosensor shown in FIG. 16A and FIG. 16B in some implementations;
FIG. 24 is a diagram of a preparation process of the preparation method for a biosensor shown in FIG. 16A and FIG. 16B in some implementations;
FIG. 25 is a schematic flowchart of step S330 of the preparation method shown in FIG. 16A and FIG. 16B in some implementations;
FIG. 26 is a diagram of a preparation process of step S330 shown in FIG. 25;
FIG. 27 is a diagram of a preparation process of the preparation method for a biosensor shown in FIG. 16A and FIG. 16B in some implementations;
FIG. 28 is a diagram of a preparation process of the preparation method for a biosensor shown in FIG. 16A and FIG. 16B in some implementations;
FIG. 29 is a diagram of a preparation process of the preparation method for a biosensor shown in FIG. 16A and FIG. 16B in some implementations; and
FIG. 30 is a diagram of structures of a detection system and a detection device in an implementation according to an implementation of this application.

### DESCRIPTION OF EMBODIMENTS

The following describes implementations of this application with reference to the accompanying drawings in implementations of this application.

In the descriptions of implementations of this application, it should be noted that, unless otherwise clearly specified and limited, the term "connection" should be understood in a broad sense. For example, the "connection" may be a direct connection, or may be an indirect connection through an intermediate medium. "A plurality of" means at least two. In implementations of this application, the terms "first", "second", "third", and "fourth" are merely intended for a purpose of description, and shall not be understood as an indication or implication of relative importance or an implicit indication of a quantity of indicated technical features. Therefore, a feature limited by "first", "second", "third", or "fourth" may explicitly or implicitly include one or more features.

Reference to "an implementation", "some implementations", or the like described in this specification means that one or more implementations of this application include a specific feature, structure, or characteristic described with reference to the implementations. Therefore, statements such as "in an implementation", "in some implementations", "in other implementations", and "in another implementation" that appear at different places in this specification do not necessarily mean referring to a same implementation. Instead, the statements mean "one or more but not all of implementations", unless otherwise specifically emphasized in another manner. The terms "include", "have", and variants of the terms all mean "include but are not limited to", unless otherwise specifically emphasized in another manner.

It can be understood that specific implementations described herein are merely used to explain a related invention, but are not intended to limit the invention. In addition, it should be noted that, for ease of description, only a part related to the invention is shown in the accompanying drawings.

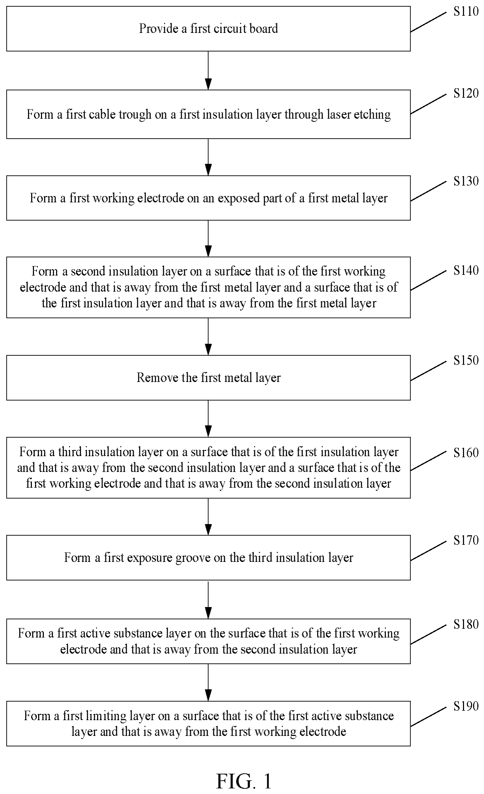

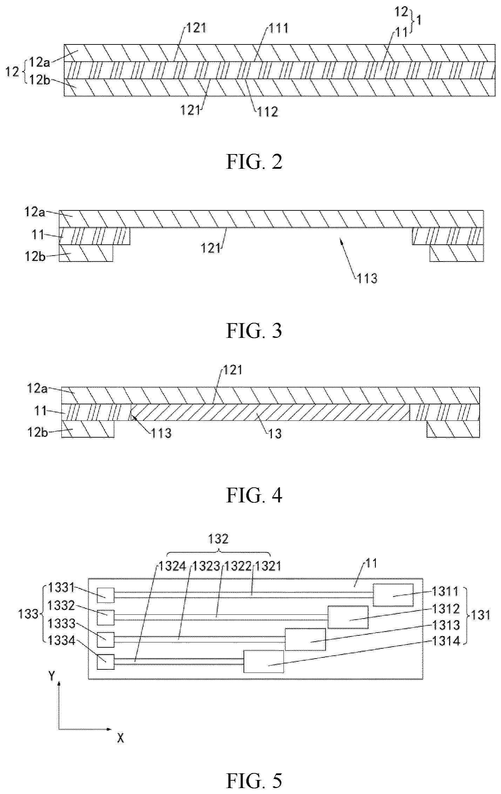

FIG. 1 is a schematic flowchart of a preparation method for a biosensor 10 in an implementation according to an implementation of this application. FIG. 2 to FIG. 15 are diagrams of a preparation process of the preparation method shown in FIG. 1 in some implementations.

As shown in FIG. 1, this implementation provides the preparation method for the biosensor 10. Steps of the preparation method may include but are not limited to step S110 to step S190 provided below. The following specifically describes the steps with reference to related accompanying drawings.

S110: Provide a first circuit board.

For example, as shown in FIG. 2, the first circuit board 1 may include a first insulation layer 11 and a first metal layer 12 that is stacked on the first insulation layer 11. A surface that is of the first metal layer 12 and that is close to the first insulation layer 11 is a first surface 121. The first insulation layer 11 may be connected to the first surface 121 of the first metal layer 12.

For example, the first circuit board 1 may be a rigid circuit board, or may be a flexible circuit board, or may be a rigid-flex circuit board. A material of the first metal layer 12 may be copper. A material of the first insulation layer 11 may be polyimide (Polyimide, PI), polyethylene terephthalate (polyethylene glycol terephthalate, PET), photosensitive adhesive, resin, or the like.

For example, there may be two first metal layers 12: a 1^{st} first metal layer 12a and a 2^{nd} first metal layer 12b. The first insulation layer 11 may include a first surface 111 and a second surface 112 that are disposed opposite to each other. The 1^{st} first metal layer 12a may be disposed on the first surface 111 of the first insulation layer 11. In this case, the first surface 121 of the 1^{st} first metal layer 12a may be connected to the first surface 111 of the first insulation layer 11. The 2^{nd} first metal layer 12b may be disposed on the second surface 112 of the first insulation layer 11. In this case, the first surface 121 of the 2^{nd} first metal layer 12b may be connected to the second surface 112 of the first insulation layer 11. In this way, the first insulation layer 11 is sandwiched between the 1^{st} first metal layer 12a and the 2^{nd} first metal layer 12b, so that the first circuit board 1 has higher structural reliability and is unlikely to bend. This is beneficial for subsequent preparation.

S120: Form a first cable trough on the first insulation layer through laser etching.

For example, as shown in FIG. 3, when the first metal layer 12 includes the 1^{st} first metal layer 12a and the 2^{nd} first metal layer 12b, before the first cable trough 113 is formed, a part of the 2^{nd} first metal layer 12b further needs to be removed through etching, to expose a part of the first insulation layer 11. Then, the first cable trough 113 is formed on the exposed part of the first insulation layer 11 through laser etching. In this case, a part of the first surface 121 of the 1^{st} first metal layer 12a may be exposed through the first cable trough 113.

For example, a radial width of the first cable trough 113 may be within a range of 10 micrometers to 100 micrometers. For example, the radial width of the first cable trough 113 may be 10 micrometers, 20 micrometers, 50 micrometers, or the like.

In some implementations, when there is one first metal layer 12, the first cable trough 113 may alternatively be formed directly on the first insulation layer 11 through laser etching. The part of the first surface 121 of the first metal layer 12 may be exposed through the first cable trough 113.

S130: Form a first working electrode on the exposed part of the first metal layer.

For example, as shown in FIG. 4 and FIG. 5, the first working electrode 1311, a first counter electrode 1312, a first reference electrode 1313, and a first blank electrode 1314 may be simultaneously formed by using an electroplating process on a part that is of the first surface 121 of the 1^{st} first metal layer 12a and that is exposed through the first cable trough 113. The first working electrode 1311, the first counter electrode 1312, the first reference electrode 1313, and the first blank electrode 1314 may jointly form a first electrode 131. In this case, the first working electrode 1311, the first counter electrode 1312, the first reference electrode 1313, and the first blank electrode 1314 may be located on a same plane. The first working electrode 1311, the first counter electrode 1312, the first reference electrode 1313, and the first blank electrode 1314 may be separated from each other by the first insulation layer 11.

For example, the first working electrode 1311, the first counter electrode 1312, the first reference electrode 1313, and the first blank electrode 1314 may be disposed on the first surface 121 in a staggered manner. For example, the first working electrode 1311, the first counter electrode 1312, the first reference electrode 1313, and the first blank electrode 1314 may be arranged on the first surface 121 in a stepped manner. In another implementation, the first working electrode 1311, the first counter electrode 1312, the first reference electrode 1313, and the first blank electrode 1314 may alternatively be arranged in another form.

In another implementation, the first working electrode 1311, the first counter electrode 1312, the first reference electrode 1313, and the first blank electrode 1314 may be located on different planes.

For example, a shape of the first working electrode 1311 may be a circle, a rectangle, a diamond, a trapezoid, a triangle, a polygon, or the like. Shapes of the first working electrode 1311, the first counter electrode 1312, the first reference electrode 1313, and the first blank electrode 1314 may be the same or may be different. In this implementation, there may be one first working electrode 1311. In another implementation, there may alternatively be a plurality of first working electrodes 1311. In another implementation, the first counter electrode 1312, the first reference electrode 1313, and the first blank electrode 1314 may alternatively be sequentially formed on the first surface 121 after the step of forming the first working electrode 1311.

As shown in FIG. 4 and FIG. 5, a first trace 132 and a first pad 133 may be further formed on the exposed part of the first metal layer 12 by using an electroplating process. The first electrode 131 may be connected to the first pad 133 through the first trace 132. The first pad 133 may be configured to connect to an external device such as a connector. In other words, the first electrode 131 may be connected to the external device through the first pad 133. The first electrode 131, the first trace 132, and the first pad 133 may jointly form a first conducting layer 13. An extension direction of the first conducting layer 13 is a first direction (namely, an X-axis direction in this implementation). A width direction of the first conducting layer 13 is a second direction (namely, a Y-axis direction in this implementation).

For example, a thickness of the first conducting layer 13 may be within a range of 100 nanometers to 20 micrometers. A radial width of the first trace 132 may be within a range of 10 micrometers to 100 micrometers. For example, the thickness of the first conducting layer 13 may be 100 nanometers, 150 nanometers, 200 nanometers, or the like. The radial width of the first trace 132 may be 10 micrometers, 20 micrometers, 50 micrometers, or the like.

As shown in FIG. 5, the first trace 132 may include a first sub-trace 1321, a second sub-trace 1322, a third sub-trace 1323, and a fourth sub-trace 1324. The first sub-trace 1321, the second sub-trace 1322, the third sub-trace 1323, and the fourth sub-trace 1324 may be separated from each other by the first insulation layer 11. For example, the first sub-trace 1321, the second sub-trace 1322, the third sub-trace 1323, and the fourth sub-trace 1324 may be sequentially arranged along the second direction.

As shown in FIG. 5, the first pad 133 may include a first sub-pad 1331, a second sub-pad 1332, a third sub-pad 1333, and a fourth sub-pad 1334. The first sub-pad 1331, the second sub-pad 1332, the third sub-pad 1333, and the fourth sub-pad 1334 may be separated from each other by the first insulation layer 11. For example, the first sub-pad 1331, the second sub-pad 1332, the third sub-pad 1333, and the fourth sub-pad 1334 may be sequentially arranged along the second direction.

For example, the first working electrode 1311 may be connected to the first sub-pad 1331 through the first sub-trace 1321. The first counter electrode 1312 may be connected to the second sub-pad 1332 through the second sub-trace 1322. The first reference electrode 1313 may be connected to the third sub-pad 1333 through the third sub-trace 1323. The first blank electrode 1314 may be connected to the fourth sub-pad 1334 through the fourth sub-trace 1324.

For example, a width of a distance between any two adjacent sub-traces in the first trace 132 may be within a range of 10 micrometers to 100 micrometers.

In another implementation, the first blank electrode 1314 may alternatively not be formed. In this case, the first trace 132 may not include the fourth sub-trace 1324. The first pad 133 may alternatively not include the fourth sub-pad 1334.

S140: Form a second insulation layer on a surface that is of the first working electrode and that is away from the first metal layer and a surface that is of the first insulation layer and that is away from the first metal layer.

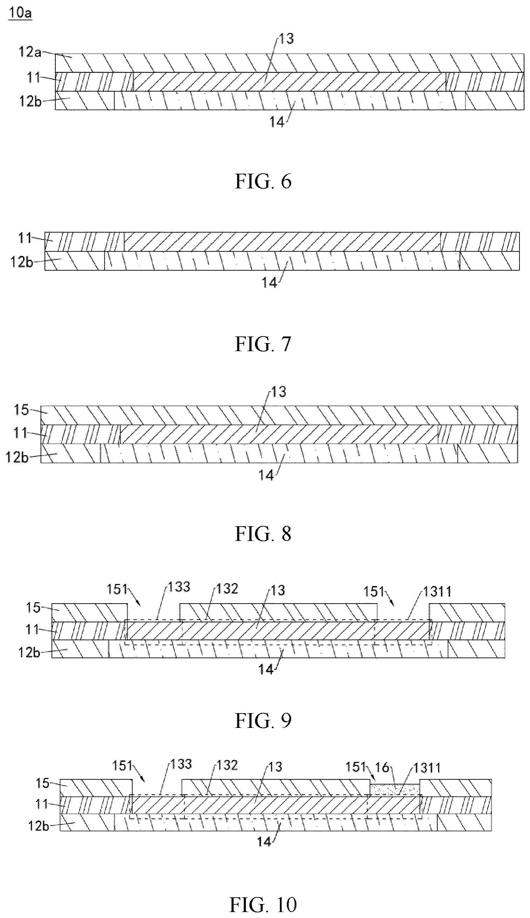

For example, as shown in FIG. 6, the second insulation layer 14 may be formed on a surface that is of the first conducting layer 13 and that is away from the 1^{st} first metal layer 12a and the surface that is of the first insulation layer 11 and that is away from the 1^{st} first metal layer 12a, to form a first sensor substrate 10a. The second insulation layer 14 may be connected to the first conducting layer 13, the first insulation layer 11, and the 2^{nd} first metal layer 12b. A material of the second insulation layer 14 may be PI, PET, photosensitive adhesive, or the like.

In some implementations, when the material of the second insulation layer 14 is PI or photosensitive adhesive, a PI solution or photosensitive adhesive may be coated on the surface that is of the first conducting layer 13 and that is away from the first metal layer 12 and the surface that is of the first insulation layer 11 and that is away from the first metal layer 12, and then the PI solution or photosensitive adhesive is cured, to form the second insulation layer 14. A coating method may be spraying, spin coating, blade coating, or the like.

S150: Remove the first metal layer.

For example, as shown in FIG. 7, the 1^{st} first metal layer 12a may be removed through etching, to expose the first conducting layer 13 and the first insulation layer 11. This facilitates subsequent preparation.

S160: Form a third insulation layer on a surface that is of the first insulation layer and that is away from the second insulation layer and a surface that is of the first working electrode and that is away from the second insulation layer.

For example, as shown in FIG. 8, the third insulation layer 15 may be formed on the surface that is of the first insulation layer 11 and that is away from the second insulation layer 14 and a surface that is of the first conducting layer 13 and that is away from the second insulation layer 14. The third insulation layer 15 may be connected to the first conducting layer 13 and the first insulation layer 11. A material of the third insulation layer 15 may be PI, PET, photosensitive adhesive, or the like. A thickness of the third insulation layer 15 may be within a range of 1 micrometer to 100 micrometers.

In some implementations, when the material of the third insulation layer 15 is PI or photosensitive adhesive, a PI solution or photosensitive adhesive may be coated on the surface that is of the first conducting layer 13 and that is away from the second insulation layer 14 and the surface that is of the first insulation layer 11 and that is away from the second insulation layer 14, and then the PI solution or photosensitive adhesive is cured, to form the third insulation layer 15. A coating method may be spraying, spin coating, blade coating, or the like.

S170: Form a first exposure groove on the third insulation layer.

For example, as shown in FIG. 9, the first exposure groove 151 may be formed through laser etching in a region that is of the third insulation layer 15 and that corresponds to the first electrode 131 and a region that is of the third insulation layer 15 and that corresponds to the first pad 133. In this case, the first exposure groove 151 may expose at least a part of the first electrode 131 and at least a part of the first pad 133. The first exposure groove 151 shown in FIG. 9 exposes the first working electrode 1311 in the first electrode 131.

For example, a shape of the first exposure groove 151 may include but is not limited to shapes such as a circle, a rectangle, a diamond, a trapezoid, a triangle, and a polygon.

In some implementations, the third insulation layer 15 having a through via may alternatively be formed in step S160, and the through via may face the first pad 133 directly, to form a part of the first exposure groove 151.

It should be noted that, for ease of understanding, dashed lines are used to show the first working electrode 1311, the first trace 132, and the first pad 133 in FIG. 9 and subsequent accompanying drawings.

S180: Form a first active substance layer on the surface that is of the first working electrode and that is away from the second insulation layer.

For example, as shown in FIG. 10, the first active substance layer 16 may be formed, in a coating manner, on a part that is of a surface of the first working electrode 1311 and that is exposed through the first exposure groove 151. In this case, the first active substance layer 16 may be connected to the surface that is of the first working electrode 1311 and that is away from the second insulation layer 14. A shape of the first active substance layer 16 may be the same as the shape of the first exposure groove 151. A thickness of the first active substance layer 16 may be different from a thickness of the first exposure groove 151 (namely, the thickness of the third insulation layer 15). In another implementation, a thickness of the first active substance layer 16 may alternatively be the same as the thickness of the first exposure groove 151.

For example, the first active substance layer 16 may be an active substance layer, such as a glucose oxidase composite layer or a lactate oxidase composite layer, that can react with a target analyte, to detect a concentration of the corresponding target analyte.

In some implementations, when there are a plurality of first working electrodes 1311, there may also be a plurality of first active substance layers 16. A quantity of first working electrodes 1311 may be the same as a quantity of first active substance layers 16. First active substance layers 16 may react with the same target analyte, or may react with different target analytes respectively. In other words, the plurality of first working electrodes 1311 in the biosensor 10 may measure concentrations of the same target analyte or may detect concentrations of different target analytes respectively.

S190: Form a first limiting layer on a surface that is of the first active substance layer and that is away from the first working electrode.

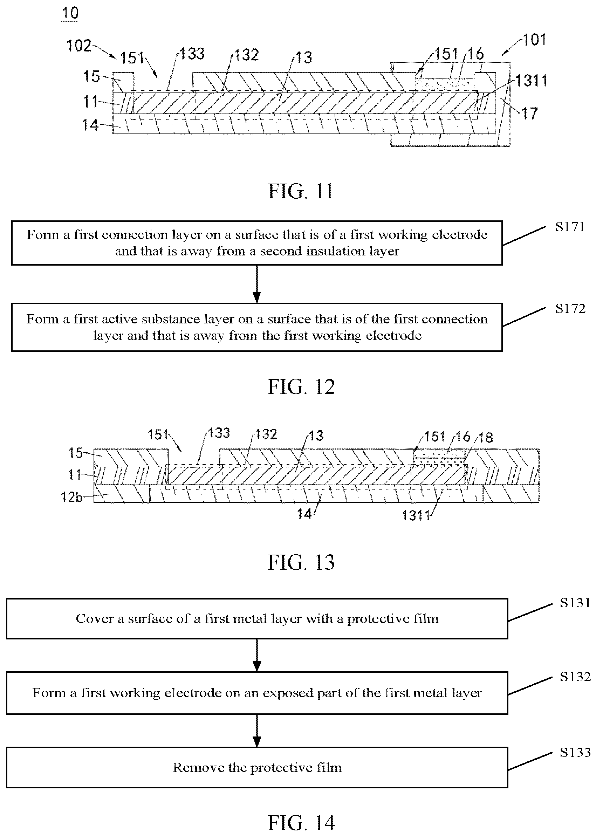

For example, as shown in FIG. 11, the first limiting layer 17 may be formed on a surface that is of the entire biosensor 10 and that is close to an end part of the first working electrode 1311 in a coating manner. The biosensor 10 is then formed through laser cutting and molding. In this case, an overall thickness of the biosensor 10 may be less than 200 micrometers. In another implementation, the first limiting layer 17 may be formed after laser cutting and molding.

For example, one end that is of the biosensor 10 and that is close to the first working electrode 1311 may form a detection end 101 of the biosensor 10. One end that is of the biosensor 10 and that is close to the first pad 133 may form a connection end 102 of the biosensor 10. The first limiting layer 17 may wrap the detection end 101 of the biosensor 10, and cover the first active substance layer 16. The first limiting layer 17 may be connected to a surface that is of the third insulation layer 15 and that is away from the first insulation layer 11, an end face that is of the third insulation layer 15 and that is close to the first working electrode 1311, an end face that is of the first insulation layer 11 and that is close to the first working electrode 1311, a surface that is of the second insulation layer 14 and that is away from the first insulation layer 11, an end face that is of the second insulation layer 14 and that is close to the first working electrode 1311, the surface that is of the first active substance layer 16 and that is away from the first working electrode 1311, and a surface that is of the first electrode 131 except the first working electrode 1311 and that is away from the second insulation layer 14.

For example, a coating method may be spraying, printing, dip coating, or the like. For example, a material of the first limiting layer 17 may be a biocompatible material such as polyvinylpyrrolidone, polyolefin, polyamide, or a related compound.

In some implementations, the first limiting layer 17 may be formed only on the surface that is of the first active substance layer 16 and that is away from the first working electrode 1311. In this case, the first limiting layer 17 may be connected to the surface that is of the first active substance layer 16 and that is away from the first working electrode 1311. Alternatively, the first limiting layer 17 may be connected to the surface that is of the first active substance layer 16 and that is away from the first working electrode 1311, and is located in the first exposure groove 151.

In this implementation, the biosensor 10 shown in FIG. 11 may be formed by using the foregoing step S110 to step S190. The biosensor 10 may include the first insulation layer 11, the second insulation layer 14, the third insulation layer 15, the first conducting layer 13, the first active substance layer 16, and the first limiting layer 17. The first insulation layer 11 may include the first surface 111 and the second surface 112 that are disposed opposite to each other. The second insulation layer 14 may be stacked on the second surface 112 of the first insulation layer 11. The third insulation layer 15 may be stacked on the first surface 111 of the first insulation layer 11. The first insulation layer 11 may be provided with the first cable trough 113. The first cable trough 113 may penetrate through the first surface 111 and the second surface 112 of the first insulation layer 11. The first conducting layer 13 may be embedded in the first cable trough 113, and is connected to the second insulation layer 14. The first conducting layer 13 may include the first electrode 131, the first trace 132, and the first pad 133. The first electrode 131 may be connected to the first pad 133 through the first trace 132. The third insulation layer 15 may be provided with the first exposure groove 151. The first exposure groove 151 may expose at least the part of the first electrode 131 and at least the part of the first pad 133. The first active substance layer 16 may be disposed in the first exposure groove 151, and is connected to a surface that is of the first electrode 131 and that is away from the first insulation layer 11. In this case, one end that is of the biosensor 10 and that is close to the first electrode 131 may form the detection end 101 of the biosensor 10. The detection end 101 may be configured to detect a concentration change of the target analyte. The end that is of the biosensor 10 and that is close to the first pad 133 may form the connection end 102 of the biosensor 10. The connection end 102 may be configured to connect to the external device such as a connector. The first limiting layer 17 may wrap the detection end 101 of the biosensor 10, and cover the first active substance layer 16.

It may be understood that, according to the biosensor 10 in this implementation, the first cable trough 113 is first etched by using a high-precision laser, and then a circuit (namely, the first conducting layer 13 in this implementation) is formed in the first cable trough 113 by using an electroplating process, so that precision of the formed circuit is high. In addition, the preparation method for the biosensor 10 provided in this implementation is different from a manner in which the biosensor 10 is prepared by using a semiconductor front-end-of-line or a micro-electro-mechanical system (Micro Electro Mechanical Systems, MEMS)-like process in that preparation is performed based on a conventional flexible circuit board (Flexible Printed Circuit, FPC) process, so that the biosensor 10 can be directly produced in a conventional FPC factory, to facilitate mass production of the biosensor 10 and improve a production capacity. In addition, the preparation method in this implementation may be compatible with a current FPC process. Preparation is directly performed on a circuit board by using both a laser etching process and an electroplating process, so that a technological process of the preparation method is simpler. In this way, cost is lower, and production efficiency is effectively improved. In addition, compared with the biosensor 10 prepared by using a printing process, the biosensor 10 prepared according to the preparation method in this implementation has higher consistency and higher precision. The width of the first trace 132 in the prepared biosensor 10 may be within the range of 10 micrometers to 100 micrometers. In other words, according to the preparation method for the biosensor 10 in this implementation, the biosensor 10 with high precision and high consistency can be obtained when a technological process is simplified, to improve a production capacity and reduce manufacturing cost.

Second, in the preparation method in this implementation, the third insulation layer 15 is further formed on the surface that is of the first insulation layer 11 and that is away from the second insulation layer 14 and a surface that is of the first electrode 131 and that is away from the second insulation layer 14, and the first exposure groove 151 is formed on the third insulation layer 15 to expose at least a part of the first electrode 131. In this way, the formed first exposure groove 151 may limit the formed first active substance layer 16 in a plane direction (namely, an X-Y plane direction in this implementation), so that an area and a shape of the first active substance layer 16 can be accurately controlled, to improve detection precision of the biosensor 10. In addition, the third insulation layer 15 may protect a part (namely, the first trace 132 in this implementation) that is of the first electrode 131 and that is not exposed through the first exposure groove 151, so that direct contact between the part that is of the first electrode and that is not exposed through the first exposure groove and an external environment is avoided, to avoid a fault such as a short circuit of the biosensor 10, and structural stability of the biosensor 10 can be improved, to improve a service life of the biosensor 10.

In addition, the biosensor 10 prepared according to the preparation method in this implementation is a four-electrode system that includes the first working electrode 1311, the first counter electrode 1312, the first reference electrode 1313, and the first blank electrode 1314. In this way, the first blank electrode 1314 is disposed, to effectively reduce interference of signal background noise, so that detection precision of the biosensor 10 is improved. In addition, the first working electrode 1311, the first counter electrode 1312, the first reference electrode 1313, and the first blank electrode 1314 are located on a same plane, so that an overall thickness of the biosensor 10 can be greatly reduced when good electrochemical performance is ensured. In this way, a thin design of the biosensor 10 is implemented, to improve wearing comfort of the biosensor 10, so that user experience is improved.

In addition, in the biosensor 10 prepared according to the preparation method in this implementation, the first limiting layer 17 is further disposed on the surface of the first active substance layer 16. In this way, the first limiting layer 17 may restrict the target analyte from entering the first active substance layer 16, so that a linear detection range of the biosensor 10 can be expanded. In addition, the material of the first limiting layer 17 is a biocompatible material, so that the detection end 101 of the biosensor 10 can be implanted into a human body, and no rejection reaction occurs when the detection end reacts with the target analyte in the human body. In this way, the biosensor can be better used in human body detection.

In some implementations, the preparation method for the biosensor 10 may alternatively not include steps S160 and S170. In this case, the first active substance layer 16 may be directly formed on the surface that is of the first working electrode 1311 and that is away from the second insulation layer 14. A shape and an area of the first active substance layer 16 may not be limited by the first exposure groove 151.

In some implementations, the preparation method for the biosensor 10 may alternatively not include step S190. In this case, the first active substance layer 16 may be in direct contact with the target substance, and react with the target analyte. In addition, the overall thickness of the biosensor 10 may be less than 100 micrometers. In this case, the biosensor 10 may be configured to directly detect a concentration of the target analyte in the external environment.

In some implementations, a part that is of a surface of the first reference electrode 1313 and that is exposed through the first exposure groove 151 may further be coated with a substance such as silver chloride, to improve detection precision. In another implementation, a surface/surfaces of the first counter electrode 1312 and/or the first blank electrode 1314 may also be coated with a corresponding substance, to improve detection precision.

FIG. 12 is a schematic flowchart of step S170 of the preparation method shown in FIG. 1 in some implementations.

In some implementations, as shown in FIG. 12, step S170 may further include but is not limited to the following steps S171 and S172.

S171: Form a first connection layer on the surface that is of the first working electrode and that is away from the second insulation layer.

For example, as shown in FIG. 13, the first connection layer 18 may be formed, in a coating manner, on the surface that is of the first working electrode 1311 and that is away from the second insulation layer 14. A material of the first connection layer 18 may be graphene, carbon nanotube, carbon, or the like.

S172: Form the first active substance layer on a surface that is of the first connection layer and that is away from the first working electrode.

For example, as shown in FIG. 13, the first active substance layer 16 may be formed, in a coating manner, on the surface that is of the first connection layer 18 and that is away from the first working electrode 1311. It may be understood that, in this implementation, the first connection layer 18 is disposed to be connected between the first active substance layer 16 and the first working electrode 1311, so that a bonding force between the first active substance layer 16 and the first working electrode 1311 can be effectively increased, to improve overall structural stability of the biosensor 10.

FIG. 14 is a schematic flowchart of step S130 of the preparation method shown in FIG. 1 in some implementations.

In some implementations, as shown in FIG. 14, step S130 may further include but is not limited to the following steps S131 to S133.

S131: Cover the surface of the first metal layer with a protective film.

S132: Form the first working electrode on the exposed part of the first metal layer.

S133: Remove the protective film.

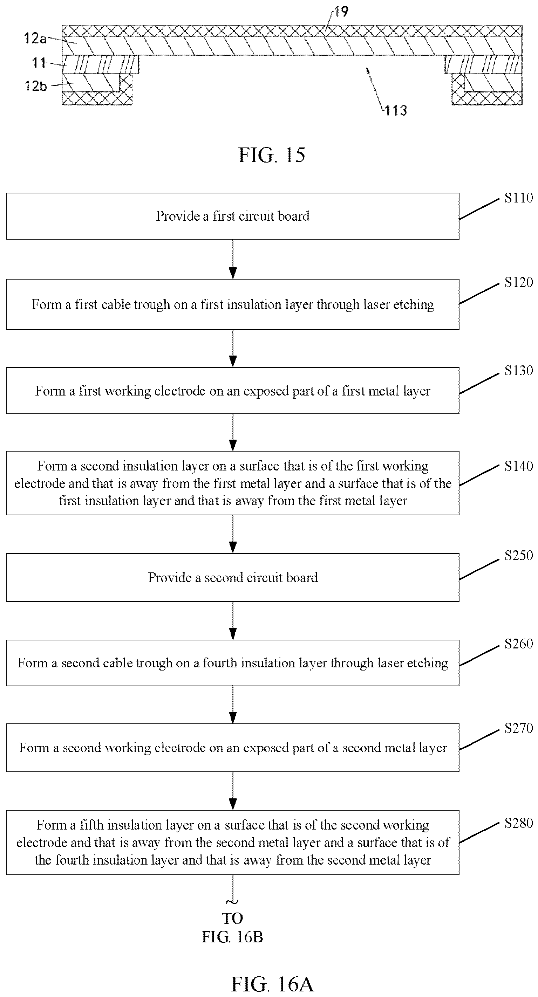

For example, as shown in FIG. 15, the protective film 19 may be covered on a surface that is of the 1^{st} first metal layer 12a and that is away from the first insulation layer 11, a surface that is of the 2^{nd} first metal layer 12a and that is away from the first insulation layer 11, and an end face that is of the 2^{nd} first metal layer 12b and that is close to the first cable trough 113. The protective film 19 may be a dry film. In this case, a part that is of the first surface 121 of the 1^{st} first metal layer 12a and that is exposed through the first cable trough 113 is not covered with the protective film 19. Finally, after the first conducting layer 13 (namely, the first working electrode 1311) is formed, the protective film 19 is removed (refer to FIG. 4).

It may be understood that, in this implementation, the first metal layer 12 and the end face that is of the first metal layer 12 and that is close to the first cable trough 113 are covered with the protective film 19, so that the first metal layer 12 can be protected in a subsequent process of forming the first working electrode 1311. In this way, a short circuit between the formed first working electrode 1311 and the first metal layer 12 is avoided, to improve electrical reliability of a product and improve a product yield.

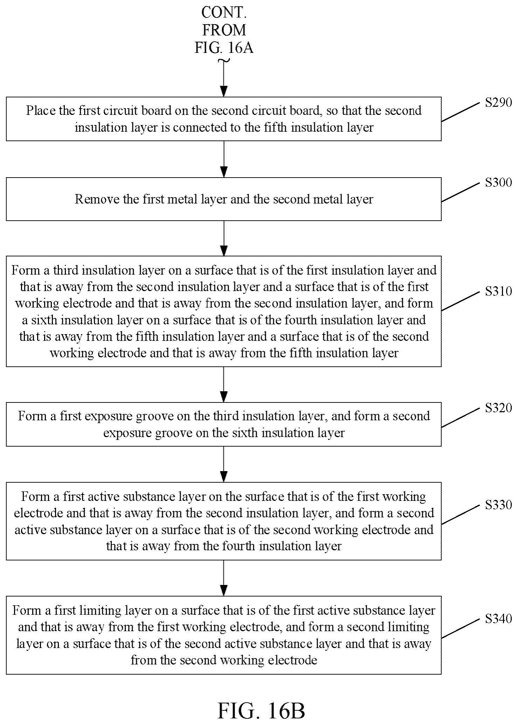

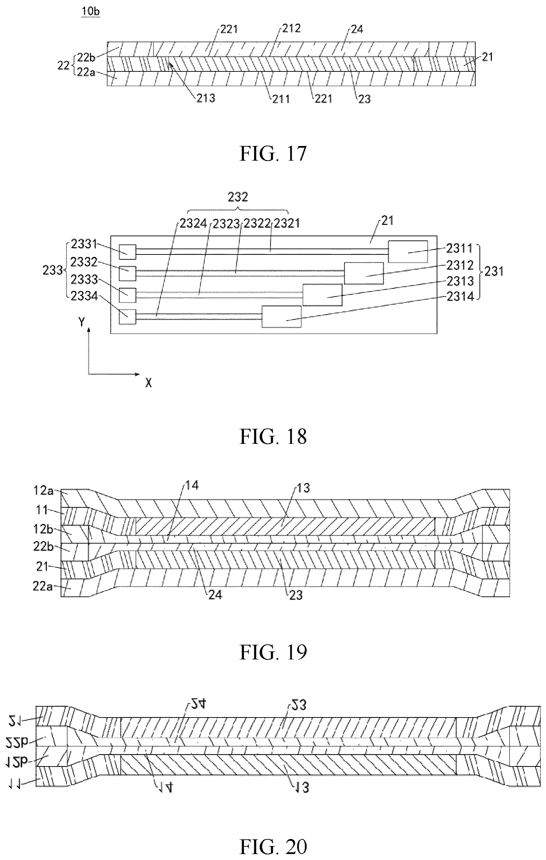

FIG. 16A and FIG. 16B are a schematic flowchart of the preparation method for the biosensor 10 shown in FIG. 1 in another implementation. FIG. 17 to FIG. 24 are diagrams of a preparation process of the preparation method for a biosensor shown in FIG. 16A and FIG. 16B in some implementations.

As shown in FIG. 16A and FIG. 16B, the preparation method for the biosensor 10 in this implementation is roughly the same as the preparation method shown in FIG. 1, and a same part is not described again. A difference lies in that, after step S140, the preparation method in this implementation may further include step S250 to step S340. The following provides specific descriptions with reference to related accompanying drawings.

S110: Provide the first circuit board.

S120: Form the first cable trough on the first insulation layer through laser etching.

S130: Form the first working electrode on the exposed part of the first metal layer.

S140: Form the second insulation layer on the surface that is of the first working electrode and that is away from the first metal layer and the surface that is of the first insulation layer and that is away from the first metal layer.

It should be understood that the first sensor substrate 10a may be prepared by using the foregoing step S110 to step S140. For step S110 to step S140, refer to step S110 to step S140 in the preparation method shown in FIG. 1. Details are not described herein again.

S250: Provide a second circuit board, where the second circuit board may include a fourth insulation layer and a second metal layer stacked on the fourth insulation layer.

S260: Form a second cable trough on the fourth insulation layer through laser etching, where a part of the second metal layer is exposed through the second cable trough.

S270: Form a second working electrode on the exposed part of the second metal layer.

S280: Form a fifth insulation layer on a surface that is of the second working electrode and that is away from the second metal layer and a surface that is of the fourth insulation layer and that is away from the second metal layer.

It should be understood that a second sensor substrate 10b may be prepared by using the foregoing step S250 to step S280. For step S250 to step S280, refer to step S110 to step S140 in the preparation method shown in FIG. 1. Details are not described herein again.

For example, as shown in FIG. 17, the second sensor substrate 10b prepared by using step S250 to step S280 may include the fourth insulation layer 21, the second metal layer 22, a second conducting layer 23, and the fifth insulation layer 24. A surface that is of the second metal layer 22 and that is close to the fourth insulation layer 21 is a second surface 221. The fourth insulation layer 21 may be connected to the second surface 221 of the second metal layer 22.

For example, there may be two second metal layers 22: a 1^{st} second metal layer 22a and a 2^{nd} second metal layer 22b. The fourth insulation layer 21 may include a first surface 211 and a second surface 212 that are disposed opposite to each other. The 1^{st} second metal layer 22a may be connected to the first surface 211 of the fourth insulation layer 21. In this case, the second surface 221 of the 1^{st} second metal layer 22a may be connected to the first surface 211 of the fourth insulation layer 21. The 2^{nd} second metal layer 22b may be connected to the second surface 212 of the fourth insulation layer. In this case, the second surface 221 of the 2^{nd} second metal layer 22b may be connected to the second surface 212 of the fourth insulation layer 21.

For example, the fourth insulation layer 21 may be provided with the second cable trough 213. The second cable trough 213 may penetrate through the first surface 211 and the second surface 212 of the fourth insulation layer 21. The second conducting layer 23 may be embedded in the second cable trough 213. The fifth insulation layer 24 may be connected to the second surface 212 of the fourth insulation layer 21, the second conducting layer 23, and the 2^{nd} second metal layer 22b.

As shown in FIG. 17 and FIG. 18, the second conducting layer 23 may include a second electrode 231, a second trace 232, and a second pad 233. The second electrode 231 may be connected to the second pad 233 through the second trace 232. The second pad 233 may be configured to connect to an external device such as a connector. In other words, the second electrode 231 may be connected to the external device through the second pad 233.

For example, a thickness of the second conducting layer 23 may be within a range of 100 nanometers to 20 micrometers. A radial width of the second trace 232 may be within a range of 10 micrometers to 100 micrometers.

For example, the second electrode 231 may include the second working electrode 2311, a second counter electrode 2312, a second reference electrode 2313, and a second blank electrode 2314. The second working electrode 2311, the second counter electrode 2312, the second reference electrode 2313, and the second blank electrode 2314 may all be formed on the second surface 221 of the 1^{st} second metal layer 22a. In this case, the second working electrode 2311, the second counter electrode 2312, the second reference electrode 2313, and the second blank electrode 2314 may be located on a same plane. The second working electrode 2311, the second counter electrode 2312, the second reference electrode 2313, and the second blank electrode 2314 may be separated from each other by the second insulation layer 14.

For example, the second working electrode 2311, the second counter electrode 2312, the second reference electrode 2313, and the second blank electrode 2314 may be disposed on the second surface 221 in a staggered manner. For example, the second working electrode 2311, the second counter electrode 2312, the second reference electrode 2313, and the second blank electrode 2314 may be arranged on the second plane in a stepped manner. In another implementation, the second working electrode 2311, the second counter electrode 2312, the second reference electrode 2313, and the second blank electrode 2314 may alternatively be arranged in another manner.

In another implementation, the second working electrode 2311, the second counter electrode 2312, the second reference electrode 2313, and the second blank electrode 2314 may be located on different planes.

It should be noted that an arrangement of electrodes in the second electrode 231 may be the same as or different from the arrangement (refer to FIG. 5) of electrodes in the first electrode 131.

For example, a shape of the second working electrode 2311 may be a circle, a rectangle, a diamond, a trapezoid, a triangle, a polygon, or the like. Shapes of the second working electrode 2311, the second counter electrode 2312, the second reference electrode 2313, and the second blank electrode 2314 may be the same or may be different. In this implementation, there may be one second working electrode 2311. In another implementation, there may alternatively be a plurality of second working electrodes 2311.

As shown in FIG. 18, the second trace 232 may include a first sub-trace 2321, a second sub-trace 2322, a third sub-trace 2323, and a fourth sub-trace 2324. The first sub-trace 2321, the second sub-trace 2322, the third sub-trace 2323, and the fourth sub-trace 2324 of the second trace 232 may be separated from each other by the second insulation layer 14. For example, the first sub-trace 2321, the second sub-trace 2322, the third sub-trace 2323, and the fourth sub-trace 2324 of the second trace 232 may be sequentially arranged along the second direction. The second pad 233 may include a first sub-pad 2331, a second sub-pad 2332, a third sub-pad 2333, and a fourth sub-pad 2334. For example, the first sub-pad 2331, the second sub-pad 2332, the third sub-pad 2333, and the fourth sub-pad 2334 of the second pad 233 may be sequentially arranged along the second direction.

For example, the second working electrode 2311 may be connected to the first sub-pad 2331 through the first sub-trace 2321. The second counter electrode 2312 may be connected to the second sub-pad 2332 through the second sub-trace 2322. The second reference electrode 2313 may be connected to the third sub-pad 2333 through the third sub-trace 2323. The second blank electrode 2314 may be connected to the fourth sub-pad 2334 through the fourth sub-trace 2324.

For example, a width of a distance between any two adjacent sub-traces in the second trace 232 may be within a range of 10 micrometers to 100 micrometers.

In another implementation, the second electrode 231 may not include the second blank electrode 2314. In this case, the second trace 232 may not include the fourth sub-trace 2324. The second pad 233 may alternatively not include the fourth sub-pad 2334.

S290: Place the first circuit board on the second circuit board, so that the second insulation layer is connected to the fifth insulation layer.

For example, as shown in FIG. 19, the first sensor substrate 10a may be placed on the second sensor substrate 10b, and pressure toward the second sensor substrate 10b is applied to the first sensor substrate 10a, so that the second sensor substrate 10b is fastened to the first sensor substrate 10a, and the second insulation layer 14 may be connected to the fifth insulation layer 24. In a press-fitting process, the second insulation layer 14 and the fifth insulation layer 24 are thinned due to extrusion pressure. In another implementation, the second insulation layer 14 may be further connected to the fifth insulation layer 24 through bonding.

In some implementations, when the arrangement of electrodes in the second electrode 231 may be the same as the arrangement of electrodes in the first electrode 131, the first electrode 131 and the second electrode 231 may be symmetrically disposed relative to the second insulation layer 14 and the fifth insulation layer 24.

S300: Remove the first metal layer and the second metal layer.

For example, as shown in FIG. 20, the 1^{st} first metal layer 12a may be removed through etching, to expose the first conducting layer 13 and the first insulation layer 11, and the 1^{st} second metal layer 22a may be removed through etching, to expose the second conducting layer 23 and the fourth insulation layer 21. This facilitates subsequent preparation.

S310: Form the third insulation layer on the surface that is of the first insulation layer and that is away from the second insulation layer and the surface that is of the first working electrode and that is away from the second insulation layer, and form a sixth insulation layer on a surface that is of the fourth insulation layer and that is away from the fifth insulation layer and a surface that is of the second working electrode and that is away from the fifth insulation layer.

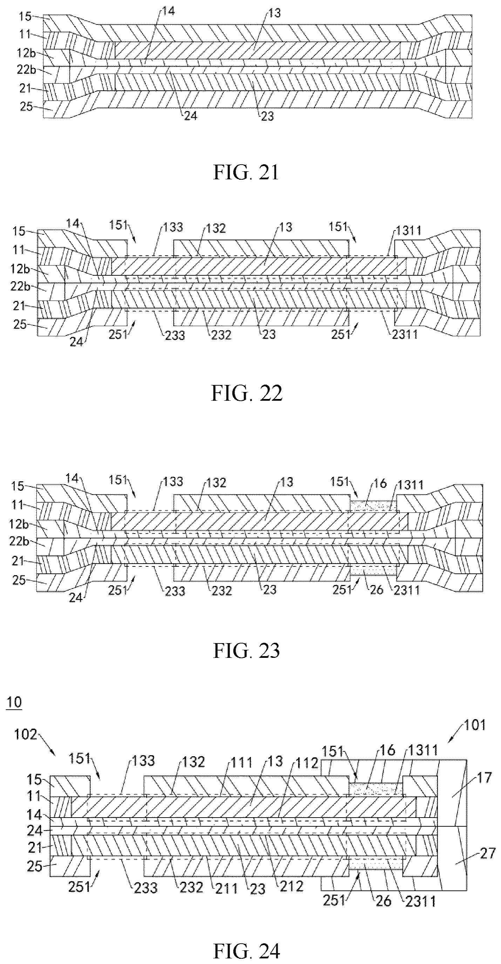

For example, as shown in FIG. 21, the third insulation layer 15 may be formed on the surface that is of the first insulation layer 11 and that is away from the second insulation layer 14 and the surface that is of the first conducting layer 13 and that is away from the second insulation layer 14. The third insulation layer 15 may be connected to the first conducting layer 13 and the first insulation layer 11. In addition, the sixth insulation layer 25 may be formed on the surface that is of the fourth insulation layer 21 and that is away from the fifth insulation layer 24 and the surface that is of the second conducting layer 23 and that is away from the fifth insulation layer 24. The sixth insulation layer 25 may be connected to the second conducting layer 23 and the fourth insulation layer 21.

Materials of both the third insulation layer 15 and the sixth insulation layer 25 may be PI, PET, photosensitive adhesive, or the like. The thickness of the third insulation layer 15 may be within the range of 1 micrometer to 100 micrometers. A thickness of the sixth insulation layer 25 may also be within the range of 1 micrometer to 100 micrometers.

S320: Form the first exposure groove on the third insulation layer, and form a second exposure groove on the sixth insulation layer.

For example, as shown in FIG. 22, the first exposure groove 151 may be formed through laser etching in the region that is of the third insulation layer 15 and that corresponds to the first electrode 131 and the region that is of the third insulation layer 15 and that corresponds to the first pad 133. In this case, the first exposure groove 151 may expose at least a part of the first electrode 131 and at least a part of the first pad 133. The first exposure groove 151 shown in FIG. 22 exposes the first working electrode 1311 in the first electrode 131.

For example, the second exposure groove 251 may be formed through laser etching in a region that is of the sixth insulation layer 25 and that corresponds to the second electrode 231 and a region that is of the sixth insulation layer 25 and that corresponds to the second pad 233. In this case, the second exposure groove 251 may expose at least a part of the second electrode 231 and at least a part of the second pad 233. The second exposure groove 251 shown in FIG. 22 exposes the second working electrode 2311 in the second electrode 231.

It should be noted that, for ease of understanding, dashed lines are used to show the first working electrode 1311, the first trace 132, the first pad 133, the second working electrode 2311, the second trace 232, and the second pad 233 in FIG. 22 and subsequent accompanying drawings.

S330: Form the first active substance layer on the surface that is of the first working electrode and that is away from the second insulation layer, and form a second active substance layer on a surface that is of the second working electrode and that is away from the fourth insulation layer.

For example, as shown in FIG. 23, the first active substance layer 16 may be formed, in a coating manner, on the part that is of the surface of the first working electrode 1311 and that is exposed through the first exposure groove 151, and the second active substance layer 26 may be formed, in a coating manner, on a part that is of the surface of the second working electrode 2311 and that is exposed through the second exposure groove 251. In this case, the first active substance layer 16 may be connected to the surface that is of the first working electrode 1311 and that is away from the second insulation layer 14. The second active substance layer 26 may be connected to a surface that is of the second working electrode 2311 and that is away from the fourth insulation layer 21. The shape of the first active substance layer 16 may be the same as the shape of the first exposure groove 151. A shape of the second active substance layer 26 may be the same as a shape of the second exposure groove 251. A thickness of the second active substance layer 26 may be different from a thickness of the second exposure groove 251 (namely, the thickness of the sixth insulation layer 25). In another implementation, the thickness of the second active substance layer 26 may alternatively be the same as the thickness of the second exposure groove 251.

For example, both the first active substance layer 16 and the second active substance layer 26 may be an active substance, such as a glucose oxidase composite layer or a lactate oxidase composite layer, that can react with the target analyte. The first active substance layer 16 and the second active substance layer 26 may react with different target analytes. In other words, the first working electrode 1311 and the second working electrode 2311 may detect concentrations of different target analytes respectively.

In another implementation, the first active substance layer 16 and the second active substance layer 26 may alternatively react with the same target analyte. In other words, the first active substance layer 16 and the second active substance layer 26 may detect concentrations of the same target analyte at different positions.

S340: Form the first limiting layer on the surface that is of the first active substance layer and that is away from the first working electrode, and form a second limiting layer on a surface that is of the second active substance layer and that is away from the second working electrode.

For example, as shown in FIG. 24, the first limiting layer 17 and the second limiting layer 27 may be separately formed on a surface of the detection end 101 of the biosensor 10 in a coating manner. Then, the biosensor 10 is formed through laser cutting and molding.

For example, the first limiting layer 17 may be connected to the surface that is of the third insulation layer 15 and that is away from the first insulation layer 11, the end face that is of the third insulation layer 15 and that is close to the first working electrode 1311, the end face that is of the first insulation layer 11 and that is close to the first working electrode 1311, the end face that is of the second insulation layer 14 and that is close to the first working electrode 1311, the surface that is of the first active substance layer 16 and that is away from the first working electrode 1311, and the surface that is of the first electrode 131 except the first working electrode 1311 and that is away from the second insulation layer 14. The second limiting layer 27 may be connected to a surface that is of the sixth insulation layer 25 and that is away from the fourth insulation layer 21, an end face that is of the sixth insulation layer 25 and that is close to the second working electrode 2311, an end face that is of the fourth insulation layer 21 and that is close to the second working electrode 2311, a surface that is of the second active substance layer 26 and that is away from the second working electrode 2311, and a surface that is of the second electrode 231 except the first working electrode 1311 and that is away from the fourth insulation layer 21.

For example, both the first working electrode 1311 and the second working electrode 2311 may be disposed at a same end of the biosensor 10. Both the first pad 133 and the second pad 233 may be disposed at a same end of the biosensor 10. In this case, one end that is of the biosensor 10 and that is close to the first working electrode 1311 and the second working electrode 2311 may form the detection end 101 of the biosensor 10. One end that is of the biosensor 10 and that is close to the first pad 133 and the second pad 233 may form the connection end 102 of the biosensor 10.

In another implementation, the first working electrode 1311 and the second working electrode 2311 may alternatively be not disposed at a same end of the biosensor 10. Alternatively, the biosensor 10 may further have a plurality of detection ends 101. This is not limited in this application.

In another implementation, the first pad 133 and the second pad 233 may alternatively be not disposed at a same end of the biosensor 10. Alternatively, the biosensor 10 may further have a plurality of connection ends 102. This is not limited in this application.

For example, a coating method may be spraying, printing, dip coating, or the like. For example, materials of both the first limiting layer 17 and the second limiting layer 27 may be polyvinylpyrrolidone, polyolefin, polyamide, or the like. The materials of the first limiting layer 17 and the second limiting layer 27 may be different. In another implementation, when the first active substance layer 16 and the second active substance layer 26 can detect a same target analyte, the materials of the first limiting layer 17 and the second limiting layer 27 may alternatively be the same.

In some implementations, the first limiting layer 17 may be formed only on the surface that is of the first active substance layer 16 and that is away from the first working electrode 1311, and the second limiting layer 27 may be formed on the surface that is of the second active substance layer 26 and that is away from the second working electrode 2311. In this case, the first limiting layer 17 may be connected to the surface that is of the first active substance layer 16 and that is away from the first working electrode 1311. The second limiting layer 27 may be connected to the surface that is of the second active substance layer 26 and that is away from the second working electrode 2311. Alternatively, the first limiting layer 17 may be connected to the surface that is of the first active substance layer 16 and that is away from the first working electrode 1311, and is located in the first exposure groove 151. The second limiting layer 27 may be connected to the surface that is of the second active substance layer 26 and that is away from the second working electrode 2311, and is located in the second exposure groove 251.

In this implementation, the biosensor 10 shown in FIG. 24 may be formed by using the foregoing step S110 to step S340. The biosensor 10 may include the first insulation layer 11, the second insulation layer 14, the third insulation layer 15, the fourth insulation layer 21, the fifth insulation layer 24, the sixth insulation layer 25, the first conducting layer 13, the second conducting layer 23, the first active substance layer 16, the second active substance layer 26, the first limiting layer 17, and the second limiting layer 27.

The first insulation layer 11 may include the first surface 111 and the second surface 112 that are disposed opposite to each other. The second insulation layer 14 may be stacked on the second surface 112 of the first insulation layer 11. The third insulation layer 15 may be stacked on the first surface 111 of the first insulation layer 11. The first insulation layer 11 may be provided with the first cable trough 113. The first cable trough 113 may penetrate through the first surface 111 and the second surface 112 of the first insulation layer 11. The first conducting layer 13 may be embedded in the first cable trough 113, and is connected to the second insulation layer 14. The first conducting layer 13 may include the first electrode 131, the first trace 132, and the first pad 133. The first electrode 131 may be connected to the first pad 133 through the first trace 132. The third insulation layer 15 may be provided with the first exposure groove 151. The first exposure groove 151 may expose at least the part of the first electrode 131 and at least the part of the first pad 133. The first active substance layer 16 may be disposed in the first exposure groove 151, and is connected to the surface that is of the first electrode 131 and that is away from the first insulation layer 11.

The fourth insulation layer 21 may include the first surface 211 and the second surface 212 that are disposed opposite to each other. The fifth insulation layer 24 may be stacked on the second surface 212 of the fourth insulation layer 21. The sixth insulation layer 25 may be stacked on the first surface 211 of the fourth insulation layer 21. The fourth insulation layer 21 may be provided with the second cable trough 213. The second cable trough 213 may penetrate through the first surface 211 and the second surface 212 of the fourth insulation layer 21. The second conducting layer 23 may be embedded in the second cable trough 213, and is connected to the fifth insulation layer 24. The second conducting layer 23 may include the second electrode 231, the second trace 232, and the second pad 233. The second electrode 231 may be connected to the second pad 233 through the second trace 232. The sixth insulation layer 25 may be provided with the second exposure groove 251. The second exposure groove 251 may expose at least a part of the second electrode 231 and at least a part of the second pad 233. The second active substance layer 26 may be disposed in the second exposure groove 251, and is connected to a surface that is of the second electrode 231 and that is away from the fourth insulation layer 21.

In this case, one end that is of the biosensor 10 and that is close to the first electrode 131 and the second electrode 231 may form the detection end 101 of the biosensor 10. The end that is of the biosensor 10 and that is close to the first pad 133 and the second pad 233 may form the connection end 102 of the biosensor 10. The connection end 102 may be configured to connect to the external device such as a connector. The first limiting layer 17 and the second limiting layer 27 may jointly wrap the detection end 101 of the biosensor 10. The detection end 101 may be configured to detect a concentration change of the target analyte. The first limiting layer 17 may cover the first active substance layer 16. The second limiting layer 27 may cover the second active substance layer 26.

It may be understood that different working electrodes (namely, the first working electrode 1311 and the second working electrode 2311) are respectively disposed on two sides of the biosensor 10 prepared according to the preparation method in this implementation. In this way, when the first working electrode 1311 and the second working electrode 2311 react with a same target analyte, a linear detection range of the biosensor 10 can be effectively expanded. When the first working electrode 1311 and the second working electrode 2311 react with different target analytes respectively, detection efficiency of the biosensor 10 can be effectively improved, so that one biosensor 10 can perform accurate detection on a plurality of target analytes simultaneously. Second, the preparation method in this implementation may be compatible with a current FPC process. Preparation is directly performed on a circuit board by using both a laser etching process and an electroplating process, so that an overall technological process is simpler. In this way, cost is lower, and production efficiency is effectively improved. In addition, the biosensor 10 prepared according to the preparation method in this implementation has higher consistency and higher precision. In other words, according to the preparation method for the biosensor 10 in this implementation, the biosensor 10 with high precision, high consistency, and high detection efficiency can be obtained when a technological process is simplified, to improve a production capacity and reduce manufacturing cost.

In some implementations, the preparation method for the biosensor 10 may alternatively not include steps S310 and S320. In this case, the first active substance layer 16 may be directly formed on the surface that is of the first working electrode 1311 and that is away from the second insulation layer 14. The second active substance layer 26 may be directly formed on the surface that is of the second working electrode 2311 and that is away from the fourth insulation layer 21.

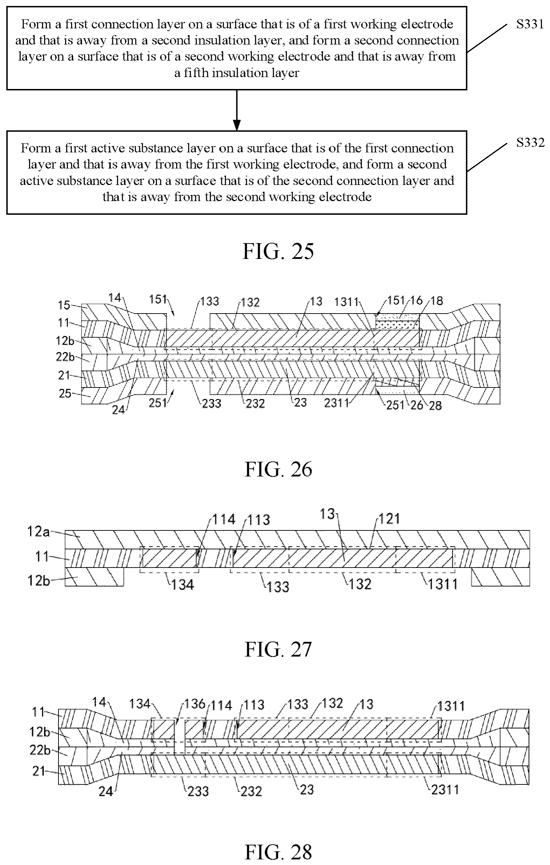

FIG. 25 is a schematic flowchart of step S330 of the preparation method shown in FIG. 16A and FIG. 16B in some implementations.

In some implementations, as shown in FIG. 25, step S330 may further include but is not limited to the following steps S331 and S332.

S331: Form the first connection layer on the surface that is of the first working electrode and that is away from the second insulation layer, and form the second connection layer on the surface that is of the second working electrode and that is away from the fifth insulation layer.

For example, as shown in FIG. 26, the first connection layer 18 may be formed, in a coating manner, on the surface that is of the first working electrode 1311 and that is away from the second insulation layer 14, and the second connection layer 28 may be formed, in a coating manner, on the surface that is of the second working electrode 2311 and that is away from the fifth insulation layer 24. Materials of both the first connection layer 18 and the second connection layer 28 may be graphene, carbon nanotube, carbon, or the like.

S332: Form the first active substance layer on the surface that is of the first connection layer and that is away from the first working electrode, and form the second active substance layer on a surface that is of the second connection layer and that is away from the second working electrode.

For example, as shown in FIG. 26, the first active substance layer 16 may be formed, in a coating manner, on the surface that is of the first connection layer 18 and that is away from the first working electrode 1311, and the second active substance layer 26 may be formed, in a coating manner, on a surface that is of the second connection layer 28 and that is away from the second electrode 231.

FIG. 27 to FIG. 29 are diagrams of a preparation process of the preparation method for the biosensor 10 shown in FIG. 16A and FIG. 16B in some implementations.

In some implementations, as shown in FIG. 27, between step S120 and step S130, a third cable trough 114 may be further formed on the first insulation layer 11. The third cable trough 114 may be separated from the first cable trough 113 by the first insulation layer 11. A part of the first surface 121 of the first metal layer 12 may further be exposed through the third cable trough 114. Between step S130 and step S140, a third pad 134 may be further formed, by using an electroplating process, on the part that is of the first surface 121 and that is exposed through the third cable trough 114. In this case, the first electrode 131, the first trace 132, the first pad 133, and the third pad 134 may jointly form the first conducting layer 13. The third pad 134 may be separated from the first electrode 131, the first trace 132, and the first pad 133 by the first insulation layer 11. In this case, the following step ① and step ② may be further included between step S300 and step S310.

Step ①: Form a first channel through laser etching sequentially performed on the third pad, the second insulation layer, and the fifth insulation layer.

For example, as shown in FIG. 28, the third pad 134 and the second pad 233 may be symmetrically disposed relative to the second insulation layer 14 and the fifth insulation layer 24. In this case, at least a part of the second pad 233 may be exposed through the first channel 136. In some implementations, a laser may further etch a small part of the second pad 233, to ensure that the first channel 136 can expose the second pads 233.

Step ②: Form a third trace on an exposed part of a surface of the second pad.

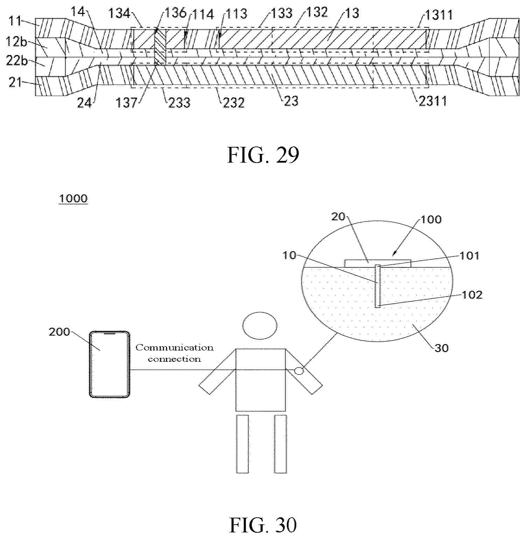

For example, as shown in FIG. 29, the third trace 137 may be formed on the exposed part of the surface of the second pad 233 by using an electroplating process. In this case, the first channel 136 may be filled with the third trace 137. The third trace 137 may be connected to the second pad 233, the second insulation layer 14, the fifth insulation layer 24, and the third pad 134. In this case, the second pad 233 may be connected to the third pad 134 through the third trace 137. In some implementations, the third trace 137 may alternatively be formed on the exposed part of the surface of the second pad 233 through electroless plating and filling with conductive metal slurry.

In the subsequent step S320, at least a part of the third pad 134 may be exposed through the formed first exposure groove 151. The formed second exposure groove 251 may alternatively not expose the second pad 233.

It may be understood that, in this implementation, the third pad 134 is further formed when the first electrode 131, the first trace 132, and the first pad 133 are formed, and is separated from the first pad 133 by the first insulation layer 11. The third pad 134 and the second pad 233 may be symmetrically disposed relative to the second insulation layer 14 and the fifth insulation layer 24. Then, the first channel 136 is formed through laser etching sequentially performed on the third pad 134, the second insulation layer 14, and the fifth insulation layer 24. Then, the third trace 137 is formed in the first channel 136, so that the second pad 233 may be connected to the third pad 134 through the third trace 137. In this way, the first electrode 131 may be electrically connected to the external environment through the first pad 133, the second electrode 231 may be electrically connected to the external environment through the third pad 134, and the first pad 133 and the third pad 134 are located on a same plane (namely, the first surface 121). In this way, when the subsequently prepared biosensor 10 is soldered or connected to another device such as the connector, connection needs to be performed on only one side of the biosensor 10, and does not need to be separately performed on two sides of the biosensor 10, so that a connection process is more convenient. In addition, when the biosensor 10 needs to be connected to the connector, the connector needs to be connected to only one side of the biosensor 10, so that an overall thickness of the biosensor 10 and the connector is thin. This helps improve user experience.

In some implementations, step S140 and step S280 may alternatively not be included. In this case, the first sensor substrate 10a may not include the second insulation layer 14. The second sensor substrate 10b may not include the fifth insulation layer 24. Then, in step S290, the first sensor substrate 10a and the second sensor substrate 10b may be bonded through an adhesive layer. After press-fitting, the adhesive layer is extruded. A part that is of the adhesive layer and that is close to the first sensor substrate 10a forms the second insulation layer 14. A part that is of the adhesive layer and that is close to the second sensor substrate 10b forms the fifth insulation layer 24. In this case, the second insulation layer 14 and the fifth insulation layer 24 may be an integrated structure.

FIG. 30 is a diagram of structures of a detection system 1000 and a detection device 100 in an implementation according to an implementation of this application.

As shown in FIG. 30, this implementation provides the detection device 100. The detection device 100 may include the biosensor 10, a connector (not shown in the figure), and a controller 20. The biosensor 10 may be prepared according to the foregoing preparation method. The connector may be connected between the connection end 102 of the biosensor 10 and the controller 20. The biosensor 10 may be electrically connected to the controller 20 through the connector.

For example, the biosensor 10 may be configured to be implanted in subcutaneous tissue 30 for detection. The detection end 101 of the biosensor 10 may be implanted in the subcutaneous tissue 30 of a human body, so that the biosensor 10 monitors in real time concentration changes of a plurality of target analytes such as blood sugar, lactic acid, ketone body, and uric acid in the human body. The controller 20 may be configured to obtain detection information detected by the biosensor 10.

As shown in FIG. 30, this implementation further provides the detection system 1000. The detection system 1000 may include the detection device 100 and an intelligent terminal 200. The intelligent terminal 200 may be a terminal having a communication function, such as a mobile phone, a computer, or a tablet computer. The controller 20 of the detection device 100 may be in communication connection with the intelligent terminal 200. In this way, when a user operates the detection device 100, and the detection end 101 of the biosensor 10 is disposed close to the target analyte, an electrode located at the detection end 101 may react with the target analyte, to obtain the detection information. Then, the controller 20 may receive the detection information, and send the detection information to the intelligent terminal 200. The intelligent terminal 200 may display the detection information to the user, to complete detection. In this way, the detection information detected by the biosensor 10 may be fed back to the intelligent terminal 200 in real time by using the controller 20, and displayed to the user, so that the user monitors a health status of the user in real time.

In some implementations, the intelligent terminal 200 may alternatively be a terminal that does not have a communication function. In this case, the controller 20 in the detection device 100 may be directly connected to the intelligent terminal 200 by using a connection cable, to implement transmission of a communication signal.

It should be noted that implementations of this application or features in implementations may be combined with each other when there is no conflict, and any combination of features in different implementations also falls within the protection scope of this application. In other words, the plurality of implementations described above may alternatively be combined according to an actual requirement.

It should be noted that all the foregoing accompanying drawings are example figures of this application, and do not represent actual sizes of products. In addition, a size proportional relationship between components in the accompanying drawings is not intended to limit an actual product in this application.

The foregoing descriptions are merely some and implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

## Claims

1. A preparation method for a biosensor (10), comprising:
providing a first circuit board (1), wherein the first circuit board (1) comprises a first insulation layer (11) and a first metal layer (12) stacked on the first insulation layer (11);
forming a first cable trough (113) on the first insulation layer (11) through laser etching, wherein a part of the first metal layer (12) is exposed through the first cable trough (113);
forming a first working electrode (1311) on the exposed part of the first metal layer (12);
forming a second insulation layer (14) on a surface that is of the first working electrode (1311) and that is away from the first metal layer (12) and a surface that is of the first insulation layer (11) and that is away from the first metal layer (12);
removing the first metal layer (12); and
forming a first active substance layer (16) on a surface that is of the first working electrode (1311) and that is away from the second insulation layer (14).

2. The preparation method according to claim 1, wherein in the step of forming the first working electrode (1311) on the exposed part of the first metal layer (12), the preparation method further comprises:
forming the first working electrode (1311) by using an electroplating process on the exposed part of the first metal layer (12).

3. The preparation method according to claim 1 or 2, wherein after the step of removing the first metal layer (12) and before the step of forming the first active substance layer (16) on the surface that is of the first working electrode (1311) and that is away from the second insulation layer (14), the preparation method further comprises:
forming a third insulation layer (15) on a surface that is of the first insulation layer (11) and that is away from the second insulation layer (14) and the surface that is of the first working electrode (1311) and that is away from the second insulation layer (14); and
forming a first exposure groove (151) on the third insulation layer (15), wherein a part of the first working electrode (1311) is exposed through the first exposure groove (151).

4. The preparation method according to any one of claims 1 to 3, wherein in the step of forming the first working electrode (1311) on the exposed part of the first metal layer (12), the preparation method further comprises:
forming the first working electrode (1311) on an exposed part of a first surface (121) of the first metal layer (12), wherein the first surface (121) is a surface that is of the first metal layer (12) and that is close to the first insulation layer (11); and
after the step of forming the first working electrode (1311) on the exposed part of the first metal layer (12), and before the step of forming the second insulation layer (14) on the surface that is of the first working electrode (1311) and that is away from the first metal layer (12) and the surface that is of the first insulation layer (11) and that is away from the first metal layer (12), the preparation method further comprises:
forming a first counter electrode (1312) and a first reference electrode (1313) on the exposed part of the first surface (121), wherein the first working electrode (1311), the first counter electrode (1312), and the first reference electrode (1313) are disposed on the first surface (121) in a staggered manner.

5. The preparation method according to claim 4, wherein after the step of forming the first working electrode (1311) on the exposed part of the first metal layer (12), and before the step of forming the second insulation layer (14) on the surface that is of the first working electrode (1311) and that is away from the first metal layer (12) and the surface that is of the first insulation layer (11) and that is away from the first metal layer (12), the preparation method further comprises:
forming a first blank electrode (1314) on the exposed part of the first surface (121), wherein the first blank electrode (1314) and the first working electrode (1311), the first counter electrode (1312), and the first reference electrode (1313) are disposed on the first surface (121) in a staggered manner.

6. The preparation method according to any one of claims 1 to 5, wherein a material of the first working electrode (1311) is platinum, gold, or palladium.

7. The preparation method according to any one of claims 1 to 6, wherein after the step of forming the first working electrode (1311) on the exposed part of the first metal layer (12), and before the step of forming the second insulation layer (14) on the surface that is of the first working electrode (1311) and that is away from the first metal layer (12) and the surface that is of the first insulation layer (11) and that is away from the first metal layer (12), the preparation method further comprises:
forming a first trace (132) and a first pad (133) on the exposed part of the first metal layer (12), wherein the first working electrode (1311) is connected to the first pad (133) through the first trace (132).

8. The preparation method according to any one of claims 1 to 7, wherein after the step of forming the second insulation layer (14) on the surface that is of the first working electrode (1311) and that is away from the first metal layer (12) and the surface that is of the first insulation layer (11) and that is away from the first metal layer (12), and before the step of removing the first metal layer (12), the preparation method further comprises:
providing a second circuit board, wherein the second circuit board comprises a fourth insulation layer (21) and a second metal layer (22) stacked on the fourth insulation layer (21);
forming a second cable trough (213) on the fourth insulation layer (21) through laser etching, wherein a part of the second metal layer (22) is exposed through the second cable trough (213);
forming a second working electrode (2311) on the exposed part of the second metal layer (22);
forming a fifth insulation layer (24) on a surface that is of the second working electrode (2311) and that is away from the second metal layer (22) and a surface that is of the fourth insulation layer (21) and that is away from the second metal layer (22); and
placing the first circuit board (1) on the second circuit board, so that the second insulation layer (14) is connected to the fifth insulation layer (24).

9. The preparation method according to claim 8, wherein in the step of removing the first metal layer (12), the preparation method further comprises:
removing the second metal layer (22); and
in the step of forming the first active substance layer (16) on the surface of the first working electrode (1311), the preparation method further comprises:
forming a second active substance layer (26) on a surface of the second working electrode (2311).

10. The preparation method according to claim 8 or 9, wherein after the step of forming the second working electrode (2311) on the exposed part of the second metal layer (22), and before the step of forming the fifth insulation layer (24) on the surface that is of the second working electrode (2311) and that is away from the second metal layer (22) and the surface that is of the fourth insulation layer (21) and that is away from the second metal layer (22), the preparation method further comprises:
forming a second trace (232) and a second pad (233) on the exposed part of the second metal layer (22), wherein the second working electrode (2311) is connected to the second pad (233) through the second trace (232).

11. The preparation method according to claim 10, wherein after the step of forming the first cable trough (113) on the first insulation layer (11) through laser etching, and before the step of forming the first working electrode (1311) on the exposed part of the first metal layer (12), the preparation method further comprises:
forming a third cable trough (114) on the first insulation layer (11) through laser etching, wherein a part of the first metal layer (12) is exposed through the third cable trough (114), and the third cable trough (114) is separated from the first cable trough (113) by the first insulation layer (11);
after the step of forming the first working electrode (1311) on the exposed part of the first metal layer (12), and before the step of forming the second insulation layer (14) on the surface that is of the first working electrode (1311) and that is away from the first metal layer (12) and the surface that is of the first insulation layer (11) and that is away from the first metal layer (12), the preparation method further comprises:
forming a third pad (134) on the first metal layer (12) exposed through the third cable trough (114); and
after the step of removing the first metal layer (12) and before the step of forming the first active substance layer (16) on the surface that is of the first working electrode (1311) and that is away from the second insulation layer (14), the preparation method further comprises:
forming a first channel (136) through laser etching sequentially performed on the third pad (134), the second insulation layer (14), and the fifth insulation layer (24), wherein a part of the second pad (233) is exposed through the first channel (136); and
forming a third trace (137) on an exposed part of a surface of the second pad (233), wherein the first channel (136) is filled with the third trace (137), and the second pad (233) is connected to the third pad (134) through the third trace (137).

12. The preparation method according to any one of claims 1 to 11, wherein in the step of forming the first active substance layer (16) on the surface that is of the first working electrode (1311) and that is away from the second insulation layer (14), the preparation method further comprises:
forming a first connection layer (18) on the surface that is of the first working electrode (1311) and that is away from the second insulation layer (14); and
forming the first active substance layer (16) on a surface that is of the first connection layer (18) and that is away from the first working electrode (1311).

13. The preparation method according to any one of claims 1 to 12, wherein after the step of forming the first active substance layer (16) on the surface that is of the first working electrode (1311) and that is away from the second insulation layer (14), the preparation method further comprises: forming a first limiting layer (17) on a surface that is of the first active substance layer (16) and that is away from the first working electrode (1311).

14. A biosensor (10), wherein the biosensor (10) is prepared according to the preparation method according to any one of claims 1 to 13.

15. A biosensor (10), comprising a first insulation layer (11), a second insulation layer (14), a first working electrode (1311), and a first active substance layer (16), wherein the first insulation layer (11) is stacked on the second insulation layer (14), the first insulation layer (11) is provided with a first cable trough (113), the first working electrode (1311) is disposed in the first cable trough (113), the first active substance layer (16) disposed on a surface that is of the first working electrode (1311) and that faces away from the second insulation layer (14), and the first cable trough (113) is formed through laser etching.

16. The biosensor (10) according to claim 15, wherein a radial width of the first cable trough (113) is in a range of 10 micrometers to 100 micrometers.

17. The biosensor (10) according to claim 15 or 16, wherein the first working electrode (1311) is formed by using an electroplating process.

18. The biosensor (10) according to any one of claims 15 to 17, wherein the biosensor (10) further comprises a first connection layer (18), and the first connection layer (18) is connected between the first active substance layer (16) and the first working electrode (1311).

19. The biosensor (10) according to claim 18, wherein a material of the first connection layer (18) is graphene, carbon nanotube, or carbon.

20. The biosensor (10) according to any one of claims 15 to 19, wherein the biosensor (10) further comprises a third insulation layer (15), the third insulation layer (15) is stacked on a surface that is of the first insulation layer (11) and that faces away from the second insulation layer (14), the third insulation layer (15) is provided with a first exposure groove (151), and a part of the first working electrode (1311) is exposed through the first exposure groove (151).

21. The biosensor (10) according to any one of claims 15 to 20, wherein the biosensor (10) further comprises a fourth insulation layer (21), a fifth insulation layer (24), a second working electrode (2311), and a second active substance layer, the fourth insulation layer (21) is stacked on the fifth insulation layer (24), the fifth insulation layer (24) is connected to the second insulation layer (14), and the fourth insulation layer (21) is provided with a second cable trough (213), the second working electrode (2311) is disposed in the second cable trough (213), and the second active substance layer is disposed on a surface that is of the second working electrode (2311) and that faces away from the fifth insulation layer (24).

22. The biosensor (10) according to claim 21, wherein the biosensor (10) further comprises a first pad (133) and a second pad (233), the first pad (133) is disposed in the first cable trough (113), the first pad (133) is electrically connected to the first working electrode (1311), the second pad (233) is disposed in the second cable trough (213), and the second pad (233) is electrically connected to the second working electrode (2311); and
the biosensor (10) further comprises a third pad (134) and a third trace (137), the first insulation layer (11) is further provided with a third cable trough (114), the third cable trough (114) and the first cable trough (113) are spaced apart, the third pad (134) is disposed in the third cable trough (114), the biosensor (10) is further provided with a first channel (136), the first channel (136) penetrates through the third pad (134), and the second insulation layer (14) and the fifth insulation layer (24), and the third trace (137) is disposed in the first channel (136), and is electrically connected to the second pad (233) and the third pad (134).

23. A detection device (100), comprising a controller (20), a connector, and the biosensor (10) according to any one of claims 15 to 22, wherein the biosensor (10) is electrically connected to the controller (20) through the connector, and the controller (20) is configured to receive detection information of the biosensor (10).

24. A detection system (1000), comprising an intelligent terminal (200) and the detection device (100) according to claim 23, wherein the intelligent terminal (200) is in communication connection with the detection device (100).
